# EUROPEAN PATENT SPECIFICATION

(11) **EP 4 255 395 B1**
(45) Date of publication and mention of the grant of the patent: **19.08.2026**
(21) Application number: 21823556.2
(22) Date of filing: 01.12.2021
(51) Int. Cl.: A61K 9/107, A61K 47/32, A61K 47/36, A61K 47/38, A61K 47/34, A61K 8/06

(54) **EMULSION COMPRISING SIZE-CONTROLLED DROPLETS**
EMULSION MIT GRÖSSENGESTEUERTEN TRÖPFCHEN
ÉMULSION COMPRENANT DES GOUTTELETTES DE TAILLE CONTRÔLÉE

(30) Priority: 01.12.2020 EP 20306476
(43) Date of publication of application: 11.10.2023
(73) Proprietor: Calyxia, 94380 Bonneuil-sur-Marne (FR)
(72) Inventor: LAVIELLE, Nicolas, 94380 Bonneuil sur Marne (FR); DEMOULIN, Damien, 94380 Bonneuil sur Marne (FR)
(74) Representative: Icosa
(86) International application number: PCT/EP2021/083838
(87) International publication number: WO 2022/117681

(56) References cited:
- US-A1- 2008 071 077
- US-A1- 2020 094 214
- SHEKARFOROUSH ELHAMALSADAT ET AL: "Soy Protein-Gum Karaya Conjugate: Emulsifying Activity and Rheological Behavior in Aqueous System and Oil in Water Emulsion", JOURNAL OF THE AMERICAN OIL CHEMISTS SOCIETY, SPRINGER, DE, vol. 93, no. 1, 16 November 2015 (2015-11-16), pages 1 - 10, XP035947522, ISSN: 0003-021X, [retrieved on 20151116], DOI: 10.1007/S11746-015-2751-Z
- MANOJ PRETIMA ET AL: "Characterization of a Polydisperse Depletion-Flocculated Emulsion", JOURNAL OF COLLOID AND INTERFACE SCIENCE, vol. 228, no. 2, August 2000 (2000-08-01), US, pages 200 - 206, XP055803685, ISSN: 0021-9797, DOI: 10.1006/jcis.2000.6936
- NI XUEWEN ET AL: "Physical stability and rheological properties of konjac glucomannan-ethyl cellulose mixed emulsions", INTERNATIONAL JOURNAL OF BIOLOGICAL MACROMOLECULES, ELSEVIER BV, NL, vol. 92, 5 July 2016 (2016-07-05), pages 423 - 430, XP029755256, ISSN: 0141-8130, DOI: 10.1016/J.IJBIOMAC.2016.07.018
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631 - 662, XP009102859, ISSN: 0193-2691, DOI: 10.1081/DIS-120015368

## Description

### FIELD OF INVENTION

The present invention relates to an emulsion comprising size-controlled droplets dispersed in a viscoelastic continuous phase.

The invention also relates to a process for the fabrication of size-controlled droplets in a viscoelastic continuous phase of an emulsion.

### BACKGROUND OF INVENTION

Emulsions are complex systems used in many industrial applications where immiscible ingredients need to be blended in a stable manner. The understanding of the mechanism of formation of the droplets during emulsion formation is essential to control the process and the size of the fabricated droplets.

One can identify two phases in an emulsion: the continuous phase and the dispersed phase. The continuous phase is the medium in which the formation of droplets take place during the emulsification and the dispersed phase is composed of droplets dispersed in the continuous phase, fabricated under shear generated by the continuous phase.

The mechanism and the scaling law of formation of droplets have been extensively studied under simple shear in laminar and turbulent flows in purely viscous continuous phases, i.e. phases that have a Newtonian behavior under shear and exhibit no elastic properties. Under shear, when the viscous pressure overcomes the Laplace pressure of the droplet, the droplet is submitted to the mechanism of elongation-fragmentation, breaking down the "mother" droplet into smaller "daughter" droplets. The size of the formed droplets varies linearly with the ratio of the Laplace pressure over the viscous pressure.

Emulsions in viscoelastic continuous phases have also been studied for emulsions comprising one or more surfactants. The state-of-the-art mentions that the viscosity of the continuous phase (η) needs to be higher than the viscosity of the dispersed phase (ηᵢ) at low shear (γ. < 100 s⁻¹), preferentially having 0.01< ηᵢ/ η <1 in order to homogeneously rupture mother droplets into daughter droplets. It is also known that the mean diameter (D) of the fabricated droplets can be calculated using the following scaling law: $D ∼ {\left({η}_{i}/η\right)}^{0.2}$

In the prior art, Shekarforoush Elhamalsadat et al., "Soy Protein-Gum Karaya Conjugate: Emulsifying Activity and Rheological Behavior in Aqueous System and Oil in Water Emulsion", Journal of the American Oil Chemists Society, vol. 93, no. 1, 16 November 2015, pages 1-10, discloses conjugating soy protein isolate (SPI) with gum karaya to obtain emulsions wherein G'>G''. Manoj Pretima et al., "Characterization of a Polydisperse Depletion-Flocculated Emulsion", Journal of Colloid and Interface Science, vol. 228, no.2, August 2000, pages 200-206, discloses polydisperse emulsions comprising droplets dispersed in a viscoelastic continuous phase. Ni Xuewen et al., "Physical stability and rheological properties of konjac glucomannan-ethyl cellulose mixed emulsions", International Journal of Biological Macromolecules, vol. 92, 5 July 2016, pages 423-430, discloses emulsions comprising ethyl cellulose oil droplets homogeneously dispersed in konjac glucomannan (KGM) water phase. Application US 2008/071077 discloses a cosmetic formulation comprising 0.01 to 5% by weight, based on the total weight of the formulation, of carboxymethyl cellulose (CMC), the CMC forming a gel, said gel having a storage modulus G' which exceeds the loss modulus G". Application US 2020/094214 discloses a method for preparing size-controlled microcapsules comprising a photopolymerization step.

WO 97/38787 discloses that monodisperse droplets are formed when a secondary emulsion is manufactured from a polydisperse viscoelastic primary emulsion in an aqueous continuous phase comprising from 20% to 40% by weight of surfactant, if the ratio between the viscous modulus (G") and the elastic modulus (G') verifies: $G " / G ′ < 100$

However, these conditions cannot be extended to any viscoelastic continuous phase and in particular to viscoelastic continuous phase without surfactant.

Indeed, emulsions in industrial products usually contain large quantities of surfactants. Surfactants are very useful during fabrication and storage to facilitate droplet formation and prevent emulsion destabilisation. However, surfactants do not contribute to the product performance during use and sometimes interact negatively with other ingredients of the product formulation. Surfactants can also cause allergic reactions in applications requiring contact with the skin. Finally, surfactants can be very costly.

There is therefore a need for improved conditions for the formation of size-controlled droplets, that are compatible with a wide range of viscoelastic phases, and in particular to viscoelastic continuous phases not comprising a surfactant.

It was found by the Applicant that it is insufficient to have G"/G' < 100 to obtain homogeneous droplet fragmentation and form size-controlled, monodisperse droplets in viscoelastic media.

The purpose of the present invention is therefore to provide conditions that apply to a broader range of viscoelastic continuous phases.

It was surprisingly found that the continuous phase should preferably belong to the predominant viscous regime, having G" > G', in order to fabricate monodisperse, size-controlled droplets. A novel relation between the ratio G''/G' and the size and size-control of the emulsified droplets was found.

### SUMMARY

This invention relates to an emulsion comprising droplets dispersed in a viscoelastic continuous phase,
said droplets having a mean diameter Dn₅₀ inferior to 100 µm, said mean diameter being measured by light scattering technique,
   wherein said droplets are size-controlled,
said viscoelastic continuous phase comprising at least one polymer in solution in at least one solvent and at a concentration ranging from 1 to 20 % by weight of said at least one polymer in respect to the total weight of the viscoelastic continuous phase,
   wherein said at least one polymer is selected from cellulose ethers, polyacrylates, polyacrylamides, polyethylene oxide, polysaccharides, gelatin, collagen, fibrin, polylysine, albumin, casein, polydimethylsiloxane, alkyl silicones, aryl silicones, alkyl aryl silicones, polyethylene glycol dimethicone, polypropylene glycol dimethicone, waxes, fatty acids, polyurethanes, polyolefins, and a mixture thereof, provided that said at least one polymer is not an alginate,
wherein said viscoelastic continuous phase presents a ratio between the viscous modulus (G") and the elastic modulus (G') ranging from 1 to 10, the viscous modulus (G") and the elastic modulus (G') being each measured by oscillatory measurements at 20°C using a rheometer at an angular frequency of 100 rad/s.

According to one embodiment, said viscoelastic continuous phase does not comprise a surfactant.

According to one embodiment, said viscoelastic continuous phase comprises exactly one polymer in solution in at least one solvent;
wherein the product **η**_{(1%)}.**C** ranges from 100 mPa.s to 100 000 mPa.s, preferably from 100 mPa.s to 1000 mPa.s;
wherein **η**_{(1%)} represents the viscosity of the polymer solution at 1% concentration by weight in respect to the total weight of the viscoelastic continuous phase and **C** represents the concentration in weight % of the polymer solution; and
wherein the **G"/G'** ratio is proportional to the product **η_{(1%)}.C** with -0.3 as exponent.

According to one embodiment, said at least one polymer is in solution in at least one solvent at a concentration ranging from 1 to 20 % by weight in respect to the total weight of the viscoelastic continuous phase.

According to one embodiment, said polymer is selected from chitosan, carboxymethyl cellulose, methyl cellulose, guar gum, konjac gum, polyacrylic acid, polyethylene oxide and a mixture thereof.

According to one embodiment, said droplets are size-controlled monodisperse droplets, and said **G"/G'** ratio is ranging from 1 to 5, preferably said **G"/G'** ratio is ranging from 1 to 3.

According to one embodiment, said droplets have a mean diameter ranging from 1 to 70 µm, preferably ranging from 5 to 20 µm.

According to one embodiment, said viscoelastic continuous phase further comprises at least one Newtonian fluid.

According to one embodiment, the dispersed phase comprises at least one crosslinkable material and optionally at least one initiator.

According to one embodiment, the dispersed phase further comprises at least one non-miscible active agent.

The invention also relates to a process for the manufacture of an emulsion according to the invention, comprising a step of submitting the dispersed droplets to an elongation-fragmentation mechanism in the viscoelastic continuous phase.

According to one embodiment, a shear rate ranging from 1 to 20000 s⁻¹ is applied during the elongation-fragmentation step, preferably from 1 to 1000 s⁻¹.

According to one embodiment, the process further comprises a step of addition of a Newtonian fluid into the viscoelastic continuous phase.

The invention also relates to a process for the fabrication of microcapsules comprising the steps of:
- manufacturing an emulsion as described herein above, and
- cross-linking the formed droplets.

According to one embodiment, the cross-linking of the formed droplets is performed by photopolymerization of said emulsion.

### DEFINITIONS

In the present invention, the following terms have the following meanings:
The term **"crosslinkable material"** refers to a material with the ability to form covalent bonds or relatively short sequences of covalent bonds in order to join two molecules together. According to one embodiment, the crosslinkable material is selected from at least one monomer or polymer or cross-linking agent and a mixture thereof.

According to the invention, the monomer or polymer is selected from monomers and polymers comprising at least one reactive function selected from the group consisting of acrylate, methacrylate, vinyl ether, N-vinyl ether, mercaptoester, thiolene, siloxane, epoxy, oxetane, urethane, isocyanate and peroxide functions. According to one embodiment, the monomer or polymer is selected from monomers and polymers carrying at least one of the above-mentioned reactive functions and further carrying at least one function selected from the group consisting of primary, secondary and tertiary alkylamine functions, quaternary amino functions, sulphate, sulphonate, phosphate, phosphonate, carboxylate, hydroxyl, halogen and a mixture thereof.

According to one embodiment, the monomer or polymer is selected from polyethers, polyesters, polyurethanes, polyanhydride, polycarbonate, polyureas, polyethylene glycols, polypropylene glycols, polyamides, polyacetals, polyimides, polyolefins, polysulfides, polydimethylsiloxanes, polysaccharides and a mixture thereof.

The term **"cross-linking agent"** refers to a compound carrying at least two reactive functions capable of cross-linking a monomer or a polymer, or a mixture thereof. According to the invention, the at least one cross-linking agent carries at least two reactive functions selected from acrylate, methacrylate, vinyl ether, N-vinyl ether, mercaptoester, thiolene, siloxane, epoxy, oxetane, urethane, isocyanate and peroxide functions.

The term **"emulsion"** refers to a colloidal system made of two non-miscible elements, for example oil and water, which by means of specific operations manages to have a macroscopically homogeneous, but microscopically heterogeneous, appearance. A first phase, being the dispersed phase, is present in the form of droplets dispersed in a second homogeneous phase, being the continuous phase. The mean diameter of the droplets can range from 10 nm to 5 mm, preferably from 100 nm to 100 µm and most preferably from 1 µm to 70 µm.

According to a preferred embodiment, the emulsion is a single emulsion, i.e. an emulsion in which the dispersed phase is a homogeneous medium. According to one embodiment, the emulsion is a double emulsion, i.e. an emulsion in which the dispersed phase droplets are themselves composed a solid or liquid phase dispersed in another liquid phase, producing double layered droplets.

The term **"elastic modulus (G')",** also known as the storage modulus, refers to the ability of a viscoelastic material to store energy under shear. The elastic modulus is measured by oscillatory measurements using a rheometer with frequency scanning at low strain, in particular at strain below 2%. Its value for the continuous phase is taken for the angular frequency corresponding to the maximum shear rate applied to the droplets during emulsification. In particular, the elastic modulus is measured by oscillatory measurements at 20°C using a rheometer, such as MCR 72 or MCR 92 (Anton Paar), equipped with a parallel plate geometry and a 1 mm gap with angular frequency scanning at strain below 2%; its value for the continuous phase is taken for ω=100 rad/s, which is the angular frequency ω corresponding to the maximum shear rate applied to the droplets during emulsification.

The term **"mean diameter of the droplets"** refers to the Dn₅₀ diameter. Dn₅₀ is the median of the number averaged size distribution of the droplets. The size distribution of the droplets, and thus the mean diameter of the droplets, may be measured by methods well known to the skilled person in the art, e.g. by a light scattering technique such as a Mastersizer 3000 equipped with a hydro SV measuring cell, or by image analysis of optical microscopy pictures, or by image analysis of electronic microscopy pictures.

The term **"non-miscible"** refers to a liquid which does not combine or blend with another liquid, or which does not combine or blend immediately with another liquid.

The term **"non-miscible active agent"** refers to an active agent in liquid or solid form which is non-miscible with other ingredients in the dispersed phase. According to one embodiment, a non-miscible active agent is dispersed within the dispersed phase, either in liquid or solid form.

Non-limiting examples of non-miscible active agent include agents selected from a crosslinker, a hardener, an organometallic or inorganometallic catalyst, a colorant, a pigment, a perfume (such as the compounds published in the list of the International Fragrance Association IFRA), a flavouring, an antioxidant, a pH adjuster, a preservative, a softener, a conditioner, an anti-discoloration agent (such as an ammonium derivative), an anti-foaming agent (such as an alcohol ethoxylate, an alkylbenzene sulfonate, a polyethylene ethoxylate, an alkylethoxysulfate or an alkylsulfate), a brightening agent, also called a colour activator (such as a stilbene derivative, a coumarin derivative, a pyrazoline derivative, a benzoxazole derivative or a naphthalimide derivative), a biologically active compound such as an enzyme, a vitamin (eg vitamin A, B, C, D, E), a protein, a lipid, a probiotic, an alpha hydroxy acid, a ceramide, a polyphenol, hyaluronic acid, a plant extract, an emollient agent, a disinfectant agent, an antibacterial agent, an antiwear agent, a lubricating agent, an anti-UV agent or a UV absorber, a pharmacologically active molecule, a medicament., a crop protection agent, a pheromone, a fertilizer, a herbicide, an insecticide, a pesticide, a fungicide, a repellent or a disinfectant for agrochemicals, a fire retardant, also called a flame retardant, (such as a brominated polyol like tetrabromobisphenol A, a halogenated or non-halogenated organophosphorus compound, a chlorinated compound, an aluminium trihydrate, an antimony oxide, a zinc borate, a red phosphorus, a melamine, or a magnesium dihydroxide), a photonic crystal, a photochromic complex, a phase change material that can absorb or release heat during phase transition and a mixture thereof.

The term **"monodisperse"** refers to a population of particles or droplets of varied size, wherein the standard deviation of the diameter distribution is lower than 25%.

The term **"Newtonian fluid"** refers to a fluid in which the viscous stresses arising from its flow, at every point, are linearly correlated to the local strain rate, i.e. the rate of change of its deformation over time. In other words, the viscosity of a Newtonian fluid is constant and independent of the shear rate applied and the elastic modulus can be neglected compared to the storage modulus. Some fluids may exhibit a complex behavior under shear, with ranges of shear rates where the viscosity is constant and ranges of shear rates where the viscosity is shear rate dependent. According to one embodiment, a fluid is considered a Newtonian fluid when its viscosity is constant at all shear rates applied during the mixing step of the emulsion. According to one embodiment, a fluid is considered a Newtonian fluid when its viscosity is constant for shear rates ranging from 0.1 s⁻¹ to 1 000 s⁻¹. According to one embodiment, a fluid is considered a Newtonian fluid when its viscosity is constant for shear rates ranging from 0.1 s⁻¹ to 500 s⁻¹. According to one embodiment, a fluid is considered a Newtonian fluid when the relative variation of its viscosity is inferior to 10% for shear rates ranging from 0.1 s⁻¹ to 200 s⁻¹.

The term **"initiator"** refers to a compound capable of initiating or accelerating a polymerization reaction.

The term **"polydisperse"** refers to a population of particles or droplets of varied size, wherein the standard deviation of the diameter distribution is superior to 25%.

Two quantities A and B are considered **"proportional"** to each other when the ratio of said two quantities is a constant, such constant bearing the appropriate unit. In particular, the ratio of said two quantities A and B remains constant when A and B simultaneously vary. The symbol "~" means "proportional".

The term **"ranging from x to y"** refers to a range that includes both x and y values as well as every value between x and y.

The term **"size-controlled"** refers to a population of particles or droplets whose average size can be predicted from the physical parameters of the emulsion using equation (1) below.

The term **"viscous modulus (G")",** also known as the loss modulus, refers to the ability of the viscoelastic material to dissipate energy under shear. The viscous modulus is measured by oscillatory measurements using a rheometer with frequency scanning at low strain, in particular at strain below 2%. Its value for the continuous phase is taken for the angular frequency corresponding to the maximum shear rate applied to the droplets during emulsification. In particular, the viscous modulus is measured by oscillatory measurements at 20°C using a rheometer, such as MCR 72 or MCR 92 (Anton Paar), equipped with a parallel plate geometry and a 1 mm gap with angular frequency scanning at strain below 2%; its value for the continuous phase is taken for ω=100 rad/s, which is the angular frequency ω corresponding to the maximum shear rate applied to the droplets during emulsification.

The term **"viscoelasticity"** refers to the property of a material which, under the effect of shear and deformation, has both the characteristics of a purely elastic material, i.e. it is capable of storing energy, and the characteristics of a purely viscous material, i.e. it is also capable of dissipating energy.

The term **"viscoelastic continuous phase"** refers to a continuous phase in an emulsion that exhibits viscoelasticity.

### DETAILED DESCRIPTION

### Emulsion

This invention relates to an emulsion comprising size-controlled droplets dispersed in a viscoelastic continuous phase, said viscoelastic continuous phase comprising at least one polymer in solution in a solvent.

According to one embodiment, the emulsion is a single emulsion or a multiple emulsion. According to one embodiment, the emulsion is a double emulsion.

### Dispersed phase

According to one embodiment, the dispersed phase comprises at least one crosslinkable material and optionally at least one initiator.

According to one embodiment, the at least one crosslinkable material is selected from at least one monomer or polymer, at least one cross-linking agent and a mixture thereof.

According to one embodiment, the monomer or polymer is selected from monomers and polymers comprising at least one reactive function selected from the group consisting of acrylate, methacrylate, vinyl ether, N-vinyl ether, mercaptoester, thiolene, siloxane, epoxy, oxetane, urethane, isocyanate and peroxide functions. According to one embodiment, the monomer or polymer is selected from monomers and polymers carrying at least one of the above-mentioned reactive functions and further carrying at least one function selected from the group consisting of primary, secondary and tertiary alkylamine functions, quaternary amino functions, sulphate, sulphonate, phosphate, phosphonate, carboxylate, hydroxyl, halogen and a mixture thereof.

According to one embodiment, the monomer or polymer is selected from polyethers, polyesters, polyurethanes, polyanhydride, polycarbonate, polyureas, polyethylene glycols, polypropylene glycols, polyamides, polyacetals, polyimides, polyolefins, polysulfides, polydimethylsiloxanes, polysaccharides and a mixture thereof. According to one embodiment, said monomer or polymer further carries at least one reactive function selected from the group consisting of acrylate, methacrylate, vinyl ether, N-vinyl ether, mercaptoester, thiolene, siloxane, epoxy, oxetane, urethane, isocyanate and peroxide functions.

Non-limiting examples of monomers or polymers include compounds selected from poly(2-(1-naphthyloxy)-ethyl acrylate), poly(2-(2-naphthyloxy)-ethyl acrylate), poly(2-(2-naphthyloxy)-ethyl methacrylate), polysorbitol dimethacrylate, polyacrylamide, poly((2-(1-naphthyloxy) ethanol), poly(2-(2-naphthyloxy) ethanol), poly(1 -chloro-2,3-epoxypropane), poly(n-butyl isocyanate), poly(N-vinyl carbazole), poly(N-vinyl pyrrolidone), poly(p-benzamide), poly(p-chlorostyrene), poly(p-methyl styrene), poly(p-phenylene oxide), poly(p-phenylene sulfide), poly(N-(methacryloxyethyl)succinimide), polybenzimidazol, polybutadiene, polybutylene terephthalate, polychloro trifluoro ethylene, polyether imide, polyether ketone, polyether sulfone, polyhydridosilsesquioxane, poly(m-phenylene isophthalamide), poly(methyl 2-acrylamido-2-methoxyacetate), poly(2-acrylamido-2-methylpropanesulfonic acid), polymono-butyl maleate, polybutylmethacrylate, poly(N-tert-butylmethacrylamide), poly(N-n-butylmethacrylamide), polycyclohexylmethacrylamide, poly(m-xylenebisacrylamide 2,3-dimethyl-1,3-butadiene,N, N-dimethylmethacrylamide), poly(n-butyl methacrylate), poly(n-butyl methacrylate), poly(cyclohexyl methacrylate), polyisobutyl methacrylate, poly(4-cyclohexylstyrene), polycyclol acrylate, polycyclol methacrylate, polydiethyl ethoxymethylene malonate, poly(2,2,2-trifluoroethyl methacrylate), poly(1,1,1-trimethylolpropane trimethacrylate), polymethacrylate, poly(N,N-dimethylaniline, dihydrazide), poly(isophthalic dihydrazine), polydimethyl benzylketal, epichlorohydrin, poly(ethyl-3-yl),3-diethoxyacrylate), poly(ethyl-3,3-dimethylacrylate), poly(ethyl vinyl ketone), poly(vinyl ethyl ketone), poly(penten-3- one), polyformaldehyde poly(diallyl acetal), polyfumaronitrile, polyglyceryl propoxy triacrylate, polyglyceryl trimethacrylate, polyglycidoxypropyltrimethoxysilane, polyglycidyl acrylate, poly(n-heptyl acrylate), poly(n-heptyl acrylic acid ester), poly(n-heptyl methacrylate), poly(3-hydroxypropionitrile), poly(2-hydroxypropyl acrylate), poly(2-hydroxypropyl methacrylate), poly(N-(methacryloxyethyl)phthalimide), poly(1,9-nonanediol diacrylate), poly(1,9-nonanediol dimethacrylate), poly(N-(n-propyl) acrylamide), poly(ortho-phthalic acid), poly(iso-phthalic acid), poly(1 ,4-benzenedicarboxylic acid), poly(1,3-benzenedicarboxylic acid), poly(phthalic acid), poly(mono-2-acryloxyethyl ester), poly-terephthalic acid, polyphthalic anhydride, polyethylene glycol diacrylate, polyethylene glycol methacrylate, polyethylene glycol dimethacrylate, poly(isopropyl acrylate), polysorbitol pentaacrylate, polyvinyl bromoacetate, polychloroprene, poly(din-hexyl silylene), poly(di-n-propyl siloxane), polydimethyl silylene, polydiphenyl siloxane, polyvinyl propionate, polyvinyl triacetoxysilane, polyvinyl tris-tert-butoxysilane, polyvinyl butyral, polyvinyl alcohol, polyvinyl acetate, polyethylene co-vinyl acetate, poly(bisphenol-A polysulfone), poly(1,3-dioxepane), poly(1,4-phenylene vinylene), poly(2,6-dimethyl-1-A-phenylene oxide), poly(4-hydroxybenzoic acid), poly(4-methyl-1-pentene), poly(4-vinyl pyridine), polymethylacrylonitrile, polymethylphenylsiloxane, polymethylsilmethylene, polymethylsilsesquioxane, poly(phenylsilsesquioxane), poly(pyromellitimide-1,4-diphenyl ether), polytetrahydrofuran, polythiophene, poly(trimethylene oxide), polyacrylonitrile, polyether sulfone, polyethylene-co-vinyl acetate, poly(perfluoroethylene propylene), poly(perfluoralkoxyl alkane), poly(styrene-acrylonitrile) and a mixture thereof.

According to one embodiment, the cross-linking agent is selected from molecules carrying at least two reactive functions selected from acrylate, methacrylate, vinyl ether, N-vinyl ether, mercaptoester, thiolene, siloxane, epoxy, oxetane, urethane, isocyanate and peroxide functions.

Non-limiting examples of cross-linking agent include compounds selected from 1,6-hexanediol diacrylate, 1,6-hexanediol dimethacrylate, polyethylene glycol dimethacrylate, 1,9-nonanediol dimethacrylate, 1,4-butanediol dimethacrylate, 2,2-bis(4-methacryloxyphenyl) propane, 1,3-butanediol dimethacrylate, 1,10-decanediol dimethacrylate, bis(2-methacryloxyethyl)N,N'-1,9-nonylene biscarbamate, 1,4-butanediol diacrylate, ethylene glycol diacrylate, 1,5-pentanediol dimethacrylate, 1,4-phenylene diacrylate, allyl methacrylate, N,N'-methylenebisacrylamide, 2,2- bis[4-(2-hydroxy-3-methacryloxypropoxy)phenyl]propane, tetraethylene glycol diacrylate, ethylene glycol dimethacrylate, diethylene glycol diacrylate, triethylene glycol diacrylate, triethylene glycol dimethacrylate, polyethylene glycol diglycidyl ether, N, N-diallylacrylamide, 2,2-bis[4-(2-acryloxyethoxy) phenyl]propane, glycidyl methacrylate, dipentaerythritol pentaacrylate, 1,1,1-trimethylolpropane triacrylate, 1,1,1-trimethylolpropane trimethacrylate, ethylenediamine tetramethacrylate, pentaerythritol triacrylate, pentaerythritol tetraacrylate, propargyl methacrylate, 2-cyanoethyl acrylate, tricyclodecane dimethanol diacrylate, hydroxypropyl methacrylate, N-acryloxysuccinimide, N-(2-hydroxypropyl)methacrylamide, N-(3-aminopropyl)methacrylamide hydrochloride, N-(t-BOC-aminopropyl)methacrylamide, 2-aminoethyl methacrylate hydrochloride, monoacryloxyethyl phosphate, o-nitrobenzyl methacrylate, acrylic anhydride, 2-(tert-butylamino)ethyl methacrylate, N,N-diallylacrylamide, glycidyl methacrylate, 2-hydroxyethyl acrylate, 4-(2-acryloxyacehoxy)-2-hydroxybenzophenone, N-(phthalimidomethyl)acrylamide, cinnamyl methacrylate and a mixture thereof.

According to one embodiment, the at least one initiator is a photo-initiator capable of initiating or accelerating a polymerization under the effect of light radiation, selected from α-hydroxyketones, α-aminoketones, aromatic ketones, thioxanthones, quinones, α-dicarbonyl derivatives, acylphosphine oxides, and a mixture thereof.

Non-limiting examples of photo-initiators include compounds selected from 2-hydroxy-2-methyl-1-phenyl-1-propanone, marketed for example under the names DAROCUR^{®} 1 173 and 4265, IRGACURE^{®} 184, 2959, and 500 by BASF, and ADDITOL^{®} CPK by CYTEC; 2-benzyl-2-dimethylamino-1 -(4-morpholinophenyl)-butanone-1, marketed for example under the names IRGACURE^{®} 907 and 369 by BASF; aromatic ketones marketed for example under the name ESACURE^{®} TZT by LAMBERTI; thioxanthones marketed for example under the name ESACURE^{®} ITX by LANMERTI; benzyldimethylketal marketed as IRGACURE^{®} 651 by BASF; bis-acylphosphine oxides (BAPO) marketed for example under the names IRGACURE^{®} 819, 1700, and 1800, DAROCUR^{®} 4265, LUCIRIN^{®} TPO, and LUCIRIN^{®} TPO-L by the company BASF; benzophenone, phenyl glyoxylates, such as phenyl glyoxylic acid methyl ester, oxime esters, such as [1-(4-phenylsulfanylbenzoyl)heptylideneamino]benzoate, sulfonium salts, iodonium salts, and oxime sulfonates.

According to one embodiment, the at least one initiator is a thermal initiator capable of initiating or accelerating a polymerization reaction under the effect of heat. Non-limiting examples of thermal initiators include azo compounds such as 2,2'-azobis(isobutyronitrile), 2,2'-azobis-2-methylbutyronitrile or 2,2'-azobis-2,4-dimethylvaleronitrile; organic peroxides such as benzoyl peroxide or dicumyl peroxide; dicyandiamide, cyclohexyl tosylate,
According to one embodiment, the at least one initiator is a redox initiator capable of initiating or accelerating a polymerization reaction by decomposition into radicals. Non-limiting examples of redox initiators include metal ion based reducing agents coupled to a peroxide, a persulfate or a disulfide and aromatic tertiary amine reducing agents coupled to a peroxide.

According to one embodiment, the dispersed phase comprises from 15% to 95% by weight of at least one crosslinkable material in respect to the total weight of the dispersed phase. According to one embodiment, the dispersed phase comprises from 5% to 30% by weight of at least one crosslinking agent in respect to the total weight of the dispersed phase. According to one embodiment, the dispersed phase comprises from 0.1% to 5% by weight of at least one initiator in respect to the total weight of the dispersed phase.

According to one embodiment, the dispersed phase further comprises at least one non-miscible active agent. According to one embodiment, the dispersed phase further comprises from 5% to 80% by weight of at least one non-miscible active agent in respect to the total weight of the dispersed phase.

According to one embodiment, the at least one non-miscible active agent is selected from a crosslinker, a hardener, an organometallic or inorganometallic catalyst, a colorant, a pigment, a perfume (such as the compounds published in the list of the International Fragrance Association IFRA), a flavouring, an antioxidant, a pH adjuster, a preservative, a softener, a conditioner, an anti-discoloration agent (such as an ammonium derivative), an anti-foaming agent (such as an alcohol ethoxylate, an alkylbenzene sulfonate, a polyethylene ethoxylate, an alkylethoxysulfate or an alkylsulfate), a brightening agent, also called a colour activator (such as a stilbene derivative, a coumarin derivative, a pyrazoline derivative, a benzoxazole derivative or a naphthalimide derivative), a biologically active compound such as an enzyme, a vitamin (eg vitamin A, B, C, D, E), a protein, a lipid, a probiotic, an alpha hydroxy acid, a ceramide, a polyphenol, hyaluronic acid, a plant extract, an emollient agent, a disinfectant agent, an antibacterial agent, an antiwear agent, a lubricating agent, an anti-UV agent or a UV absorber, a pharmacologically active molecule, a medicament., a crop protection agent, a pheromone, a fertilizer, a herbicide, an insecticide, a pesticide, a fungicide, a repellent or a disinfectant for agrochemicals, a fire retardant, also called a flame retardant, (such as a brominated polyol like tetrabromobisphenol A, a halogenated or non-halogenated organophosphorus compound, a chlorinated compound, an aluminium trihydrate, an antimony oxide, a zinc borate, a red phosphorus, a melamine, or a magnesium dihydroxide), a photonic crystal, a photochromic complex, a phase change material that can absorb or release heat during phase transition and a mixture thereof.

According to one embodiment, the proportion of dispersed phase is between 5% and 50%, by weight in respect to the total weight of the emulsion.

According to one embodiment, the viscosity (ηᵢ) of the dispersed phase at 25°C is between 20 mPa.s and 50 000 mPa.s. According to one embodiment, the viscosity of the dispersed phase is between 20 mPa.s and 40 000 mPa.s. According to one embodiment, the viscosity of the dispersed phase is between 100 mPa.s and 40 000 mPa.s. According to one embodiment, the viscosity of the dispersed phase is between 20 mPa.s and 3 000 mPa.s. According to one embodiment, the viscosity of the dispersed phase is between 3 000 mPa.s and 40 000 mPa.s. According to one embodiment, the viscosity of the dispersed phase is between 10 000 mPa.s and 40 000 mPa.s.

The viscosity of the dispersed phase may be measured by methods well-known to a skilled person in the art, e.g. by means of a Anton Paar rheometer MCR 92 equipped with a 25 mm diameter parallel plate geometry or a 50 mm cone plate geometry with a 2-degree angle, and a temperature control cell set at 25°C. The viscosity value is read at a shear rate of 0.1 s⁻¹.

### Continuous phase

The continuous phase is a viscoelastic continuous phase. The viscoelastic continuous phase comprises at least one polymer in solution in at least one solvent at a concentration ranging from 1 to 20 % of said at least one polymer by weight in respect to the total weight of the viscoelastic continuous phase.

According to one embodiment, the solution of at least one polymer represents at least 50% of the viscoelastic continuous phase. According to one embodiment, the solution of at least one polymer represents at least 60% of the viscoelastic continuous phase. According to one embodiment, the solution of at least one polymer represent at least 70% of the viscoelastic continuous phase. According to one embodiment, the solution of at least one polymer represents at least 80% of the viscoelastic continuous phase. According to one embodiment, the solution of at least one polymer represents at least 90% of the viscoelastic continuous phase. According to one embodiment, the solution of at least one polymer represents at least 95% of the viscoelastic continuous phase. According to a preferred embodiment, the solution of at least one polymer represents 100% of the viscoelastic continuous phase.

According to one embodiment, the viscoelastic continuous phase comprises at least one polymer with a molecular weight greater than 5 000 g.mol⁻¹. According to one embodiment, the viscoelastic continuous phase comprises at least one polymer with a molecular weight between 10 000 g.mol⁻¹ and 500 000 g.mol⁻¹; preferably, between 20 000 g.mol⁻¹ and 300 000 g.mol⁻¹; most preferably between 50 000 g.mol⁻¹ and 200 000 g.mol⁻¹.

The at least one polymer is selected from the following polymers and a mixture thereof:
- cellulose ethers, e.g. methyl cellulose, ethyl cellulose, hydroxyethyl cellulose, methylhydroxyethyl cellulose, ethylhydroxyethyl cellulose, carboxymethyl cellulose, hydroxypropyl cellulose or methylhydroxypropyl cellulose;
- polyacrylates (also called carbomers), such as polyacrylic acid (PAA), polymethacrylic acid (PMAA), poly(hydroxyethyl methacrylate) (pHEMA) or poly(N-2-hydroxypropyl methacrylate) (pHPMA);
- polyacrylamides such as poly(N-isopropylacrylamide) (NIPAM); polyvinylpyrrolidone (PVP) and its derivatives or polyvinyl alcohol (PVA) and its derivatives;
- polyethylene oxide;
- polysaccharides such as carrageenan, locust bean gum or tara gum, dextran, xanthan gum, chitosan, agarose, hyaluronic acids, gellan gum, guar gum, gum arabic, tragacanth gum, diutane gum, oat gum, karaya gum, ghatti gum, curdlan gum, pectin, konjac gum or starch;
- gelatin, collagen, fibrin, polylysine, albumin or casein;
- polydimethylsiloxane (also called dimethicone), alkyl silicones, aryl silicones, alkyl aryl silicones, polyethylene glycol dimethicones or polypropylene glycol dimethicone;
- waxes such as diester waxes (alkanediol diesters, hydroxylacid diesters), triester waxes (triacylglycerols, alkane-1,2-diol triesters, ω-hydroxy acid and fatty acid triesters), esters of hydroxymalonic acid, fatty acid and alcohol, triesters of hydroxylacids, fatty acid and fatty alcohol, triesters of fatty acid, hydroxylacid and diol or polyester waxes (fatty acid polyesters);
- fatty acids (which can for example be used as waxes) such as cerotic acid, palmitic acid, stearic acid, dihydroxystearic acid, behenic acid, lignoceric acid, arachidic acid, myristic acid, lauric acid, tridecyclic acid, pentadecyclic acid, margaric acid, non-adecyclic acid, phenicosylic acid, tricosylic acid, pentacosylic acid, heptacosylic acid, montanic acid or nonacosylic acid;
- polyurethanes; and
- polyolefins such as polyisobutene.

Suitable components that can be used as waxes may be fatty acids esters such as cetyl palmitate, cetyl octanoate, cetyl laurate, cetyl lactate, cetyl isononanoate, cetyl stearate, stearyl stearate, myristyl stearate, cetyl myristate, isoscetyl stearate, glyceryl trimyristate, glyceryl tripalmitate, glyceryl monostearate or glyceryl cetyl palmitate.

According to a preferred embodiment, the at least one polymer is selected from the group consisting of cellulose ethers, polyacrylates, polysaccharides, polydimethylsiloxane, alkyl silicones, aryl silicones, alkyl aryl silicones, polyethylene glycol dimethicone, polypropylene glycol dimethicone and a mixture thereof.

According to a preferred embodiment, the at least one polymer is selected from the group consisting of cellulose ethers, polyacrylates, polyacrylamides, polysaccharides, gelatin, collagen, fibrin, polylysine, albumin, casein, polydimethylsiloxane, alkyl silicones, aryl silicones, alkyl aryl silicones, polyethylene glycol dimethicone, polypropylene glycol dimethicone and a mixture thereof.

According to a preferred embodiment, the at least one polymer is selected from the group consisting of cellulose ethers, polyacrylates, polyacrylamides, polyethylene oxide, polysaccharides, gelatin, collagen, fibrin, polylysine, albumin, casein, polydimethylsiloxane, alkyl silicones, aryl silicones, alkyl aryl silicones, polyethylene glycol dimethicone, polypropylene glycol dimethicone and a mixture thereof.

According to a more preferred embodiment, the at least one polymer is selected from the group consisting of chitosan, carboxymethyl cellulose, methyl cellulose, guar gum, konjac gum, polyacrylic acid, polyethylene oxide and a mixture thereof.

According to a more preferred embodiment, the at least one polymer is selected from the group consisting of chitosan, carboxymethyl cellulose, methyl cellulose, guar gum, konjac gum, polyacrylic acid and a mixture thereof.

The at least one polymer is not an alginate. Alginates are salts of alginic acid (algin), a natural linear polymer based on two monomeric units, β-D-mannuronic acid (M) and α-L-guluronic acid (G), covalently linked together by β(1-4) glycosidic bonds. The molar ratio M/G can vary widely as a function of the algal source, for example from 0.2 to 4. Most alginates are extracted from brown seaweeds, in particular from Laminaria, Macrocystis or Ascophyllum. The alginate salts of interest are sodium alginate, calcium alginate, ammonium alginate and potassium alginate.

According to one embodiment, said viscoelastic continuous phase does not comprise a surfactant.

Surfactants are molecules well known by the skilled artisan to stabilize an emulsion by slowing down or stopping the mechanisms of emulsion ageing such as coalescence and growth. Surfactants are selected from anionic surfactants such as alkali metal alkylbenzenesulphonates, alkali metal alkylsulphates such as sodium dodecyl sulphate, alkali metal alkyl ether sulphates, alkali metal alkylaryl ether sulphates and alkali metal dioctyl sulphosuccinates; cationic surfactants such as dialkyl (C₁₀-C₃₀) benzyldimethylammonium halides and polyethoxylated quaternary ammonium salts; amphoteric surfactant such aN-alkyl (C₁₀-C₂₂) betaines, N-alkyl (C₁₀-C₂₂) amidobetaines, (C₁₀-C₂₂)-alkyl imidazolines and asparagine derivatives; amphoteric surfactants such as alkylamphoacetates; or non-ionic surfactants such as oxyethylenated and oxypropylenated block polymers (poloxamers), polyethoxylated fatty acids, sorbitan esters, polyethoxylated sorbitan esters, polyethoxylated alkylphenols, polyethoxylated fatty alcohols, derivatives of polyethylene glycol and of a mixture of glycerides (mono-, di- and tri-glycerides) of fatty acids; polyethylene glycol derivatives of a mixture of glycerides (alkoxylated triglycerides) of vegetable origin, alkylpolyglycosides, polyethoxylated or polyglycerol fatty amides, polyglycerol alcohols and alphadiols.

According to one embodiment, said viscoelastic continuous phase comprises exactly one polymer in solution in at least one solvent.

According to one embodiment, the at one solvent is selected from the group consisting of water; alcohols such as methanol, ethanol, 1-propanol, 2-propanol, 1-butanol, t-butanol, hexanol, octanol, phenol, glycerol; ketones such as acetone, methylethyl ketone; low molecular weight carboxylic acids such as acetic acid; low molecular weight esters such as ethyl acetate; glycols such as ethylene glycol; propylene carbonate; acetonitrile; dimethylsulfoxide; and tetrahydrofurane.

According to one embodiment, the viscosity (η) of the viscoelastic continuous phase at 25°C is higher than the viscosity (ηᵢ) of the dispersed phase at 25°C. According to the one embodiment, the viscosity of the viscoelastic continuous phase at 25°C is between 2 000 mPa.s and 10 000 000 mPa.s. According to one embodiment, the viscosity of the viscoelastic continuous phase is between 5 000 mPa.s and 1 000 000 mPa.s. According to one embodiment, the viscosity of the viscoelastic continuous phase is between 10 000 mPa.s and 500 000 mPa.s.

Advantageously, the high viscosity of the viscoelastic continuous phase allows a kinetic stability of the emulsion.

The viscosity of the viscoelastic continuous phase may be measured by methods well known to the skilled person in the art, e.g. by means of a Anton Paar rheometer MCR 92 equipped with a 25 mm diameter parallel plate geometry or a 50 mm cone plate geometry with a 2-degree angle, and a temperature control cell set at 25°C. The viscosity value is read at a shear rate of 0.1 s⁻¹.

The viscoelastic continuous phase is characterized by the ratio between the viscous modulus (G") and the elastic modulus (G'), whose variation will impact the behavior of the viscoelastic continuous phase and will allow to define the domains in which no droplets, monodisperse or polydisperse size-controlled droplets are formed.

The ratio between the viscous modulus (G") and the elastic modulus (G') ranges from 1 to 10, leading to the formation of monodisperse size-controlled droplets. According to a preferred embodiment, the ratio between the viscous modulus (G") and the elastic modulus (G') ranges from 1 to 5, leading to the formation of monodisperse size-controlled droplets. According to a more preferred embodiment, the ratio between the viscous modulus (G") and the elastic modulus (G') ranges from 1 to 3, leading to the formation of monodisperse size-controlled droplets.

According to one embodiment, the ratio between the viscous modulus (G") and the elastic modulus (G') ranges from 3 to 5, leading to the formation of monodisperse size-controlled droplets. According to a preferred embodiment, the ratio between the viscous modulus (G") and the elastic modulus (G') ranges from 3 to 10, leading to the formation of monodisperse size-controlled droplets. According to a more preferred embodiment, the ratio between the viscous modulus (G") and the elastic modulus (G') ranges from 5 to 10, leading to the formation of monodisperse size-controlled droplets.

Advantageously, the variation of the diameter (D) of the formed droplets is function of the G'/G'' ratio at the angular frequency corresponding to the shear applied during emulsification. According to one embodiment, the mean diameter D of said droplets varies linearly as function of equation (1) in the domain 0.2 < G"/G' < 10. $D ∼ {\left(G′/G"\right)}^{1.5}$

The droplets have a mean diameter inferior to 100 µm. According to one embodiment, the droplets have a mean diameter ranging from 1 to 70 µm. According to one embodiment, the droplets have a mean diameter ranging from 20 to 70 µm. According to a preferred embodiment, the droplets have a mean diameter ranging from 1 to 20 µm.

According to another embodiment, when said viscoelastic continuous phase comprises exactly one polymer in solution in at least one solvent, the degree of entanglements of the polymer chains in solution can be characterized and said degree of entanglements is correlated with the G''/G' ratio. A higher degree of entanglement leads to an increase of the elastic modulus (G') of the viscoelastic continuous phase. The degree of entanglements depends on the molecular weight of the polymer and its concentration (C) in weight % in the solvent. The intrinsic viscosity, depending only on the molecular weight of the polymer, can be approximated by η_{(1%)}, which is the viscosity of the polymer solution having a polymer concentration of 1% by weight in respect to the total weight of the viscoelastic continuous phase. η_{(1%)} is intrinsic to the chosen polymer for a given solvent.

According to one embodiment, the product η_{(1%)}.C is used to characterize the degree of entanglements of the polymer solution. According to one embodiment, the G''/G' ratio depends on the degree of entanglements of the polymer chains in the solution, whose variation will allow to define the domains in which no droplets, monodisperse or polydisperse size-controlled droplets are formed.

Advantageously, the G"/G' ratio varies linearly as function of equation (2): $G " / G ′ ∼ {\left({η}_{\left(1%\right)}.C\right)}^{- 0.3}$

According to one embodiment, the product η_{(1%)}.C ranges from 100 mPa.s to 100 000 mPa.s, in this range polydisperse and monodisperse size-controlled droplets are formed.

According to a preferred embodiment, the product η_{(1%)}.C ranges from 100 mPa.s to 1 000 mPa.s, in this range monodisperse size-controlled droplets are formed.

The droplets are size-controlled monodisperse droplets.

According to one embodiment, a population of monodisperse droplets has a standard deviation of the diameter distribution less than 80%. According to one embodiment, a population of monodisperse droplets has a standard deviation of the diameter distribution less than 50%. According to a preferred embodiment, a population of monodisperse droplets has a standard deviation of the diameter distribution less than 25%. The standard deviation of the diameter distribution may be measured by methods well known to the skilled person in the art, e.g. by a light scattering technique such as a Mastersizer 3000 equipped with a hydro SV measuring cell, or by image analysis of optical microscopy pictures, or by image analysis of electronic microscopy pictures.

According to one embodiment, the viscoelastic continuous phase further comprises at least one Newtonian fluid. The at least one Newtonian fluid is either a pure ingredient or an ingredient dissolved in a solvent that is miscible with the continuous phase solvents.

Advantageously, the at least one Newtonian fluid allows a skilled artisan to control the G"/G' ratio and thus the mean diameter D of the droplets.

According to one embodiment, the viscoelastic continuous phase comprises at least one Newtonian fluid in solution at a proportion ranging from 0.05 to 50% by weight in respect to the total weight of the continuous phase. According to one embodiment, the viscoelastic continuous phase comprises at least one Newtonian fluid in solution at a proportion ranging from 0.1 to 20%. According to one embodiment, the viscoelastic continuous phase comprises at least one Newtonian fluid in solution at a proportion ranging from 0.1 to 5%. According to one embodiment, the viscoelastic continuous phase comprises at least one Newtonian fluid in solution at a proportion ranging from 20 to 50%.

According to one embodiment, the G"/G' ratio varies linearly as function of the concentration of the at least one Newtonian fluid comprised into the viscoelastic continuous phase.

According to one embodiment, the at least one Newtonian fluid is selected from vegetable oils such as avocado oil, canola oil, grape seed oil, nuts oil, olive oil, rapeseed oil, rice bran oil, safflower oil, sesame oil, soybean oil, sunflower oil; glycerol and glycerol derivatives; mineral oils; polyurethanes, polyacrylates, polyethers and cellulose ethers have a linear chain and a molecular weight ranging from 5 000 to 100 000 g/mol, preferably from 10 000 to 50 000 g/mol and most preferably from 15 000 to 30 000 g/mol, such as ethoxylate urethane polymers, hydroxyethyl cellulose; and a mixture thereof.

### Process

The invention also relates to a process for the fabrication of an emulsion as described herein above, comprising the step of mixing the emulsion, thus submitting to shear a population of droplets dispersed in the viscoelastic continuous phase, said viscoelastic continuous phase comprising at least one polymer in solution in at least one solvent.

The embodiments defining the above emulsion apply *mutatis mutandis* to the above process.

According to one embodiment, the viscosity of the continuous phase (η) is higher than the viscosity of the dispersed phase (ηᵢ), preferably, the ηᵢ/η ratio belongs to the range 0.01< ηᵢ/ η <1 in order to homogeneously rupture mother droplets into smaller daughter droplets under shear.

As described herein above, it was found by the applicant that the variation of the G"/G' ratio will impact the behavior of the viscoelastic continuous phase and will allow to define the domain in which no droplets, monodisperse or polydisperse size-controlled droplets are formed.

According to one embodiment, the ratio between the viscous modulus (G") and the elastic modulus (G') belongs to the domains as described above for the viscoelastic continuous phase, in which monodisperse droplets are formed and the mean diameter D of said droplets varies linearly as function of equation (1) mentioned above in the domain ranging from 1 to 10.

The formed droplets have a mean diameter inferior to 100 µm. According to one embodiment, the formed droplets have a mean diameter ranging from 1 to 70 µm. According to one embodiment, the formed droplets have a mean diameter ranging from 20 to 70 µm. According to a preferred embodiment, the formed droplets have a mean diameter ranging from 1 to 20 µm.

As described above, the G''/G' ratio is correlated with the degree of entanglements (η_{(1%)}.C) of the polymer chains in solution. It was found that the G"/G' ratio varies linearly as function of equation (2) mentioned above and that its variation allows to define the domain in which no droplets, monodisperse or polydisperse size-controlled droplets are formed, based on the ability to fragmentize the mother droplets.

According to one embodiment, the product η_{(1%)}.C belongs to the domains as described above, in which monodisperse size-controlled droplets are formed.

According to one embodiment, shear is applied to the emulsion in order to fragmentize the dispersed droplets. According to one embodiment, a shear rate ranging from 1 to 20 000 s⁻¹ is applied. According to a preferred embodiment, a shear rate ranging from 1 to 1 000 s⁻¹ is applied.

Formed droplets are size-controlled, monodisperse droplets.

According to one embodiment, the formed population of monodisperse droplets has a standard deviation of the diameter distribution less than 80%. According to one embodiment, the formed population of monodisperse droplets has a standard deviation of the diameter distribution less than 50%. According to a preferred embodiment, the formed population of monodisperse droplets has a standard deviation of the diameter distribution less than 25%.

According to one embodiment, the process further comprises the addition of at least one Newtonian fluid in solution into the viscoelastic continuous phase at a concentration ranging from 0.05 to 50 % by weight in respect to the total weight of the continuous phase. Advantageously, the addition of at least one Newtonian fluid allows to control the G"/G' ratio and thus the mean diameter D of the formed droplets as the G"/G' ratio varies linearly as function of the concentration of the Newtonian fluid comprised into the viscoelastic continuous phase.

According to one embodiment, the mean diameter of the formed droplets is measured just after the shear is applied. The mean diameter is measured by light scattering technique such as a Mastersizer 3000 equipped with a hydro SV measuring cell. Other methods well known to the skilled person in the art to measure a mean diameter are by image analysis of optical microscopy pictures, or by image analysis of electronic microscopy pictures.

Due to the transient nature of the emulsion (especially if the continuous phase contains no surfactant), ageing mechanisms start to broaden the size distribution of the formed droplets after the mixing is stopped. Such ageing mechanisms are well-known to the skilled artisan and include for example coalescence and growth.

According to one embodiment, the process further comprises a step of cross-linking the formed droplets as soon as mixing is stopped. Advantageously, it allows to freeze the system in order to measure the exact mean diameter of the formed droplets that is resulting from the application of a given shear. The mean diameter is measured by light scattering technique such as a Mastersizer 3000 equipped with a hydro SV measuring cell. Other methods well known to the skilled person in the art to measure a mean diameter are by image analysis of optical microscopy pictures, or by image analysis of electronic microscopy pictures.

According to one embodiment, cross-linking is performed by subjecting the emulsion to a polymerization step. According to one embodiment, cross-linking is performed by subjecting the emulsion to a photopolymerization consisting in exposing the emulsion to a light source capable of initiating the polymerization of the formed droplets. According to one embodiment, the light source is a source of UV light with a wavelength ranging from 100 nm to 400 nm. According to one embodiment, cross-linking is performed by subjecting the emulsion to a thermal polymerization consisting in exposing the emulsion to heat.

### Use of the emulsion

The emulsion as described hereinabove may be used in applications requiring size-controlled monodisperse droplets.

The emulsion as described hereinabove may be used in formulations requiring size-controlled monodisperse droplets. The emulsion as described herein above may be used in formulations requiring limited or no use of surfactants.

The emulsion of the present invention may be used in the cosmetic and personal care field, for example, in formulation of washing hygiene products, such as shampoos, shower gels, toothpastes, shaving creams or formulations of beauty products such as face and body creams, solar creams or make-up.

The emulsion of the present invention may be used in the home and fabric care field, for example, in detergent compositions for household or professional use, such as detergents, softeners, dishwashing products or cleaning products.

The emulsion of the present invention may be used in the pharmaceutical field.

The emulsion of the present invention may be used in agriculture and crop protection, for example, in phytosanitary compositions, such as antiseptics, herbicides, insecticides or fertilizers.

The emulsion of the present invention may be used in the field of paints, coatings, adhesives and sealants.

The emulsion of the present invention may be used in the field of lubricants or fuels.

The emulsion of the present invention may be used in the field of materials, plastics, composites and foams.

If a step of cross-linking of the formed droplets is performed, the formed size-controlled microparticles may be used for the encapsulation of various non-miscible active agents as per the definition above.

### BRIEF DESCRIPTION OF THE DRAWINGS

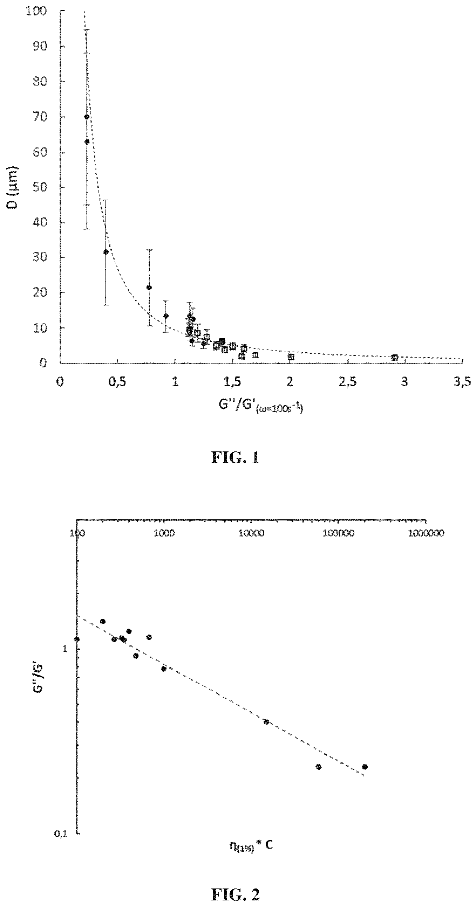

**Figure 1** is a graph showing the variation of the mean diameter (D) and size distribution of the formed droplets as a function of G''/G' ratio at the working angular frequency for materials of Table 1 (filled circles) and Table 2 (open squares). Data points correspond to the mean diameter (D) and error bars correspond to the standard deviation of the size distribution. The dotted line corresponds to the equation (1).
**Figure 2** is a graph showing the evolution of the G''/G' ratio as a function of the product η_{(1%)}.C for the materials of Table 1. The dotted line corresponds to the equation (2).

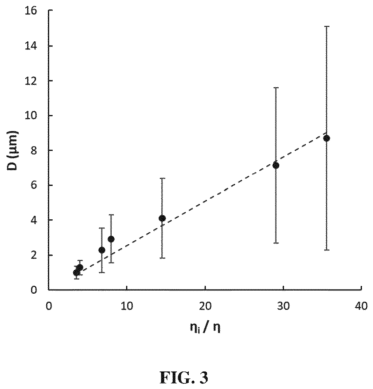

**Figure 3** is a graph showing the variation of the mean diameter (D) and size distribution of the formed droplets as a function of the ratio ηᵢ/η for the samples in Table 2. Data points correspond to the mean diameter (D) and error bars correspond to the standard deviation of the size distribution. The dotted line corresponds to equation (3).

### EXAMPLES

The present invention is further illustrated by the following examples.

All percentages mentioned for the compositions in the examples are weight percentages in respect to the total weight of the composition.

In all following examples, a dispersed phase composed of polyester polymer (CN2035, Sartomer) 92%, hexanediol diacrylate 5% and Darocur 1173 (Aldrich) 3% was used. This mixture is Newtonian with ηᵢ = 32000 mPa.s.

An emulsion made of 5% of this dispersed phase in various types of continuous phases was formed by applying a 500 s⁻¹ shear rate for one minute using a sawtooth impeller. Immediately after the shear rate was stopped, the formed droplets were cross-linked under UV light in order to freeze the system before ageing such as coalescence and growth could occur. It was therefore possible to measure the size distribution of the formed droplets as it is right after the application of shear, by means of image analysis of microscopy pictures of the cross-linked emulsions.

The monodispersity / polydispersity and size control of the formed droplets as a function of the continuous phase properties were observed and compared.

### Example 1: Droplets formation in viscoelastic media (0.2 < G"/G' < 10)

A variety of polymers solubilised in water were used as continuous phases, whose nature and properties are summarised in Table 1.

### Results

These polymer systems enable the formation of droplets with a mean diameter D ranging from 5 to 70 µm. These droplets are size-controlled as the mean diameter can be predicted from equation (1) mentioned above. The size distribution is found to be polydisperse when G''/G' is below 0.2 and monodisperse when G''/G' is above 0.2.

**Figure 1** **(filled circles)** shows the variation of the mean diameter (D) and size distribution of the formed droplets as a function of G''/G' ratio at the working angular frequency for materials of Table 1. Data points correspond to the mean diameter (D) and error bars correspond to the standard deviation of the size distribution. The black dotted line corresponds to the equation (1).

Figure 1 shows that:
- If 0.2 < G"/G' < 1, a polydisperse population of size-controlled droplets is formed.
- If 1 < G"/G' < 10, a monodisperse population of size-controlled droplets is formed by an efficient elongation-fragmentation mechanism with a typical diameter below 20 µm.

Moreover, it was found that the ratio G''/G' varies with the product η_{(1%)}.C verifying equation (2) mentioned above.

**Figure 2** shows the evolution of the G''/G' ratio as a function of the product η_{(1%)}.C for the materials of Table 1. The grey dotted line corresponds to the equation (2).

Figure 2 shows that:
- If η_{(1%)}.C <1 000 mPa.s, a monodisperse population of size-controlled droplets is formed and the continuous phase belongs to the predominant viscous regime.
- If 1 000 mPa.s < η_{(1%)}.C < 100 000 mPa.s, a polydisperse population of size-controlled droplets is formed.

**Table 1**

| **Materials** | **Supplier** | **Chemistry** | **η_{(1%)} (mPa.s)** | **C (%)** | **η_{(1%)}.C** | **G"/G'_{(ω=100 rad/s)}** |
|---|---|---|---|---|---|---|
| Konjac 36000 | Roeper | Konjac | 90 000 | 1 | 90 000 | 0.23 |
| Carbomer 141 | Adara | Polyacrylic acid | 40 000 | 1.5 | 60 000 | 0.23 |
| CMC 4570 | Roeper | Carboxymethyl cellulose | 20 000 | 1,5 | 30 000 | 0.4 |
| CMC 4520 | Roeper | Carboxymethyl cellulose | 200 | 5 | 1000 | 0.78 |
| CMC 4500 100-200 | Roeper | Carboxymethyl cellulose | 60 | 8 | 480 | 0.92 |
| Polyox WSR 205 | Dow | Polyethylene oxyde | 70 | 5 | 350 | 1.12 |
| Chitosan | Chitosan lab | Chitosan | 20 | 5 | 100 | 1.13 |
| CMC 4500 20-50 | Roeper | Carboxymethyl cellulose | 20 | 13,5 | 270 | 1.13 |
| Chitosan | Chitosan lab | Chitosan | 33 | 10 | 330 | 1.15 |
| CMC 4520 | Roeper | Carboxymethyl cellulose | 200 | 3.4 | 680 | 1.16 |
| Methocel A4C | Dow | Methyl cellulose | 80 | 5 | 400 | 1.25 |
| Chitosan | Chitosan tab | Chitosan | 20 | 10 | 200 | 1.41 |

### Example 2: Droplet formation in Newtonian fluids (G"/G' > 10)

A variety of polymers were used as continuous phases with Newtonian behavior (G"/G' > 10): hydrophobically modified ethoxylate-urethane copolymer (Optiflo T1000 and Optiflo L1400, Byk) and hydrophobically modified polyacetal-polyether copolymer (Aquaflow NHS300E, Ashland). The composition and viscosity of the different samples made are summarised in Table 2.

**Table 2**

| **Composition** | **η (mPa.s)** |
|---|---|
| Aquaflow NHS300E 58% | 900 |
| Water 42% | |
| Optiflo T1000 70% | 1 100 |
| Water 30% | |
| Aquaflow NHS300E 75% | 2 200 |
| Water 25% | |
| Optiflo L1400 80% | 4 000 |
| Water 20% | |
| Optiflo L1400 85% | 4 700 |
| Water 15% | |
| Optiflo L1400 | 8 000 |
| Aquaflow NHS300E | 9 000 |

### Results

If G"/G' > 10, the polymer solution behaves like a purely viscous Newtonian fluid. Thus, the mean diameter D does not depend on the G''/G' ratio and the size distribution remain broad.

All prepared continuous phases have a purely viscous behavior, and the diameter D of the formed droplets verifies equation (3), in which σ is the surface tension, η the viscosity of the continuous phase and γ the shear rate. In particular, D does not depend on the G''/G' ratio: $D ∼ σ / \left({ηγ}^{.}\right)$

The size distribution of the droplet population was found to be polydisperse.

**Figure 3** shows the variation of the mean diameter (D) and size distribution of the formed droplets as a function of the ratio ηᵢ/η for the samples in Table 2. Data points correspond to the mean diameter (D) and error bars correspond to the standard deviation of the size distribution. The dotted line corresponds to equation (3).

### Example 3: Insufficient fragmentation to form droplets (G"/G' < 0.2)

Two different polymers solubilised in water were used as continuous phases, whose nature and properties are summarised in Table 3.

**Table 3**

| **Materials** | **Supplier** | **Chemistry** | **η_{(1%)} (mPa.s)** | **C (%)** | **η_{(1%)}.C** | **G"/G'_{(ω=100 rad/s)}** |
|---|---|---|---|---|---|---|
| Guar 3500 | Roeper | Guar | 20 000 | 5 | 100 000 | 0.15 |
| CMC 4570 | Roeper | Carboxymethyl cellulose | 20 000 | 6 | 120 000 | 0.18 |

### Results

In these systems wherein G''/G' < 0.2 and η_{(1%)}.C > 100 000 mPa.s no droplets could be formed, showing that the elastic modulus is too high for efficient fragmentation.

### Example 4: Addition of a Newtonian fluid in the continuous phase

Different mixtures of a chitosan (Chitosanlab) solution at 5% in water and Optiflo L1400 (Byk) were used as continuous phases. Their composition and properties are summarised in Table 4.

### Results

These polymer systems enable the formation of droplets with a mean diameter D below 10 µm. These droplets are size-controlled as the mean diameter can be predicted from equation (1) mentioned above. The size distribution is found to be monodisperse which is consistent with the fact that G''/G' is above 0.2. These results can be visualised in **Figure 1** (open squares).

Moreover, it is found that G''/G' increases linearly with the concentration of the Newtonian fluid.

**Table 4**

| **Materials** | **Chitosan 5% Conc. (%)** | **Optiflo L1400 Conc. (%)** | **G"/G'_{(ω=100 rad/s)}** |
|---|---|---|---|
| 1 | 100 | 0 | 1.13 |
| 2 | 97,5 | 2,5 | 1.2 |
| 3 | 95 | 5 | 1.28 |
| 4 | 90 | 10 | 1.36 |
| 5 | 80 | 20 | 1.43 |
| 6 | 60 | 40 | 1.58 |

## Claims

1. Emulsion comprising droplets dispersed in a viscoelastic continuous phase,
said droplets having a mean diameter Dn₅₀ inferior to 100 µm, said mean diameter being measured by light scattering technique,
wherein said droplets are size-controlled,
said viscoelastic continuous phase comprising at least one polymer in solution in at least one solvent and at a concentration ranging from 1 to 20 % by weight of said at least one polymer in respect to the total weight of the viscoelastic continuous phase,
wherein said at least one polymer is selected from cellulose ethers, polyacrylates, polyacrylamides, polyethylene oxide, polysaccharides, gelatin, collagen, fibrin, polylysine, albumin, casein, polydimethylsiloxane, alkyl silicones, aryl silicones, alkyl aryl silicones, polyethylene glycol dimethicone, polypropylene glycol dimethicone, waxes, fatty acids, polyurethanes, polyolefins, and a mixture thereof, provided that said at least one polymer is not an alginate,
wherein said viscoelastic continuous phase presents a ratio between the viscous modulus **(G")** and the elastic modulus **(G')** ranging from 1 to 10, the viscous modulus (G") and the elastic modulus (G') being each measured by oscillatory measurements at 20°C using a rheometer at an angular frequency of 100 rad/s.

2. The emulsion according to claim **1,** wherein said viscoelastic continuous phase does not comprise a surfactant.

3. The emulsion according to claim **1** or **2,** wherein said polymer is selected from chitosan, carboxymethyl cellulose, methyl cellulose, guar gum, konjac gum, polyacrylic acid, polyethylene oxide and a mixture thereof.

4. The emulsion according to any one of claims **1** to **3,** wherein said droplets are size-controlled monodisperse droplets, and wherein said **G"/G'** ratio is ranging from 1 to 5, preferably said **G"/G'** ratio is ranging from 1 to 3.

5. The emulsion according to any one of claims **1** to **4,** wherein said droplets have a mean diameter ranging from 1 to 70 µm, preferably ranging from 5 to 20 µm.

6. The emulsion according to any one of claims **1** to **5,** wherein said viscoelastic continuous phase further comprises at least one Newtonian fluid.

7. The emulsion according to any one of claims **1** to **6,** wherein the dispersed phase comprises at least one crosslinkable material and optionally at least one initiator.

8. The emulsion according to claim **7,** wherein the dispersed phase further comprises at least one non-miscible active agent.

9. Process for the manufacture of an emulsion according to any one of claims **1** to **8,** comprising a step of submitting the dispersed droplets to an elongation-fragmentation mechanism in the viscoelastic continuous phase.

10. The process according to claim **9,** wherein a shear rate ranging from 1 to 20000 s⁻¹ is applied during the elongation-fragmentation step, preferably from 1 to 1000 s⁻¹.

11. The process according to claim **9** or claim **10,** further comprising a step of addition of a Newtonian fluid into the viscoelastic continuous phase.

12. Process for the fabrication of microcapsules comprising the steps of:
- manufacturing an emulsion as described in any one of claims **9** to **11,** and
- cross-linking the formed droplets.

13. The process according to claim **12,** wherein the cross-linking of the formed droplets is performed by photopolymerization of said emulsion.

## Patentansprüche

1. Emulsion, die Tröpfchen umfasst, die in einer viskoelastischen kontinuierlichen Phase dispergiert sind, wobei die Tröpfchen einen mittleren Durchmesser Dn₅₀ von weniger als 100 µm aufweisen, wobei der mittlere Durchmesser durch eine Lichtstreuungsmethode gemessen wird,
wobei die Tröpfchen größenkontrolliert sind,
wobei die viskoelastische kontinuierliche Phase mindestens ein Polymer in Lösung in mindestens einem Lösungsmittel und in einer Konzentration von 1 bis 20 Gew.-% des mindestens einen Polymers im Verhältnis zum Gesamtgewicht der viskoelastischen kontinuierlichen Phase umfasst,
wobei das mindestens eine Polymer aus Celluloseethern, Polyacrylaten, Polyacrylamiden, Polyethylenoxid, Polysacchariden, Gelatine, Kollagen, Fibrin, Polylysin, Albumin, Kasein, Polydimethylsiloxan, Alkylsilikonen, Arylsilikonen, Alkylarylsilikonen, Polyethylenglykoldimethicon, Polypropylenglykoldimethicon, Wachsen, Fettsäuren, Polyurethanen, Polyolefinen und einer Mischung davon ausgewählt ist, sofern das mindestens eine Polymer kein Alginat ist,
wobei die viskoelastische kontinuierliche Phase ein Verhältnis zwischen dem Viskositätsmodul **(G")** und dem Elastizitätsmodul **(G')** von 1 bis 10 aufweist, wobei der Viskositätsmodul (G") und der Elastizitätsmodul (G') jeweils durch oszillierende Messungen bei 20 °C mit einem Rheometer bei einer Winkelfrequenz von 100 rad/s gemessen werden.

2. Emulsion nach Anspruch **1,** wobei die viskoelastische kontinuierliche Phase kein Tensid umfasst.

3. Emulsion nach Anspruch **1** oder **2,** wobei das Polymer aus Chitosan, Carboxymethylcellulose, Methylcellulose, Guarkernmehl, Konjakgummi, Polyacrylsäure, Polyethylenoxid und einer Mischung davon ausgewählt ist.

4. Emulsion nach einem der Ansprüche **1** bis **3,** wobei es sich bei den Tröpfchen um größenkontrollierte monodisperse Tröpfchen handelt, und wobei das Verhältnis **G"/G'** von 1 bis 5 beträgt, wobei das Verhältnis **G"/G'** vorzugsweise von 1 bis 3 beträgt.

5. Emulsion nach einem der Ansprüche **1** bis **4,** wobei die Tröpfchen einen mittleren Durchmesser von 1 bis 70 µm, vorzugsweise von 5 bis 20 µm, aufweisen.

6. Emulsion nach einem der Ansprüche **1** bis **5,** wobei die viskoelastische kontinuierliche Phase ferner mindestens eine newtonsche Flüssigkeit umfasst.

7. Emulsion nach einem der Ansprüche **1** bis **6,** wobei die dispergierte Phase mindestens ein vernetzbares Material und optional mindestens einen Initiator umfasst.

8. Emulsion nach Anspruch **7,** wobei die dispergierte Phase ferner mindestens einen nicht mischbaren Wirkstoff umfasst.

9. Verfahren zur Herstellung einer Emulsion nach einem der Ansprüche **1** bis **8,** umfassend einen Schritt des Unterziehens der dispergierten Tröpfchen einem Dehnungs-Fragmentierungsmechanismus in der viskoelastischen kontinuierlichen Phase.

10. Verfahren nach Anspruch **9,** wobei während des Dehnungs-Fragmentierungsschritts eine Scherrate von 1 bis 20000 s⁻¹, vorzugsweise von 1 bis 1000 s⁻¹, angewendet wird.

11. Verfahren nach Anspruch **9** oder Anspruch **10,** ferner umfassend einen Schritt der Zugabe einer newtonschen Flüssigkeit in die viskoelastische kontinuierliche Phase.

12. Verfahren zur Herstellung von Mikrokapseln, umfassend die folgenden Schritte:
- Herstellen einer Emulsion nach einem der Ansprüche **9** bis **11,** und
- Vernetzen der gebildeten Tröpfchen.

13. Verfahren nach Anspruch **12,** wobei das Vernetzen der gebildeten Tröpfchen durch Photopolymerisation der Emulsion erfolgt.

## Revendications

1. Émulsion comprenant des gouttelettes dispersées dans une phase continue viscoélastique,
lesdites gouttelettes ayant un diamètre moyen Dn50 inférieur à 100 µm, ledit diamètre moyen étant mesuré par la technique de diffusion de la lumière,
dans laquelle lesdites gouttelettes sont de taille contrôlée,
ladite phase continue viscoélastique comprenant au moins un polymère en solution dans au moins un solvant et à une concentration allant de 1 à 20 % en poids dudit au moins un polymère par rapport au poids total de la phase continue viscoélastique,
dans laquelle ledit au moins un polymère est choisi parmi les éthers de cellulose, les polyacrylates, les polyacrylamides, l'oxyde de polyéthylène, les polysaccharides, la gélatine, le collagène, la fibrine, la polylysine, l'albumine, la caséine, le polydiméthylsiloxane, les silicones alkyliques, les silicones aryliques, les silicones alkylaryliques, le polyéthylène glycol diméthicone, le polypropylène glycol diméthicone, les cires, les acides gras, les polyuréthanes, les polyoléfines et un mélange de ceux-ci,
à condition que ledit au moins un polymère ne soit pas un alginate,
dans laquelle ladite phase continue viscoélastique présente un rapport entre le module visqueux (G") et le module élastique (G') allant de 1 à 10, le module visqueux (G") et le module élastique (G') étant chacun mesurés par des mesures oscillatoires à 20 °C à l'aide d'un rhéomètre à une fréquence angulaire de 100 rad/s.

2. L'émulsion selon la revendication **1,** dans laquelle ladite phase continue viscoélastique ne comprend pas d'agent tensioactif.

3. L'émulsion selon la revendication 1 ou 2, dans laquelle ledit polymère est choisi parmi le chitosane, la carboxyméthylcellulose, la méthylcellulose, la gomme de guar, la gomme de konjac, l'acide polyacrylique, l'oxyde de polyéthylène et un mélange de ceux-ci.

4. L'émulsion selon l'une quelconque des revendications 1 à 3, dans laquelle lesdites gouttelettes sont des gouttelettes monodispersées à taille contrôlée, et dans laquelle ledit rapport G"/G' va de 1 à 5, de préférence ledit rapport G"/G' va de 1 à 3.

5. L'émulsion selon l'une quelconque des revendications 1 à 4, dans laquelle lesdites gouttelettes ont un diamètre moyen allant de 1 à 70 µm, de préférence allant de 5 à 20 µm.

6. L'émulsion selon l'une quelconque des revendications 1 à 5, dans laquelle ladite phase continue viscoélastique comprend en outre au moins un fluide newtonien.

7. L'émulsion selon l'une quelconque des revendications 1 à 6, dans laquelle la phase dispersée comprend au moins un matériau réticulable et, éventuellement, au moins un initiateur.

8. L'émulsion selon la revendication 7, dans laquelle la phase dispersée comprend en outre au moins un agent actif non miscible.

9. Procédé de fabrication d'une émulsion selon l'une quelconque des revendications 1 à 8, comprenant une étape consistant à soumettre les gouttelettes dispersées à un mécanisme d'allongement-fragmentation dans la phase continue viscoélastique.

10. Le procédé selon la revendication 9, dans lequel un taux de cisaillement allant de 1 à 20 000 s⁻¹ est appliqué pendant l'étape d'allongement-fragmentation, de préférence allant de 1 à 1 000 s⁻¹.

11. Le procédé selon la revendication 9 ou la revendication 10, comprenant en outre une étape d'ajout d'un fluide newtonien dans la phase continue viscoélastique.

12. Procédé de fabrication de microcapsules comprenant les étapes suivantes :
- préparation d'une émulsion telle que décrite dans l'une quelconque des revendications 9 à 11, et
- réticulation des gouttelettes formées.

13. Le procédé selon la revendication 12, dans lequel la réticulation des gouttelettes formées est réalisée par photopolymérisation de ladite émulsion.
